(19)

Europäisches
Patentamt
European
Patent Office
Office européen
des brevets

(11) **EP 1 917 908 A1**

(12) **EUROPEAN PATENT APPLICATION**

(43) Date of publication:
**07.05.2008 Bulletin 2008/19**

(51) Int Cl.:
***A61B 5/103*** *(2006.01)*

(21) Application number: **07254185.7**

(22) Date of filing: **22.10.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **03.11.2006 GB 0621963**

(71) Applicant: **Astron Clinica Limited**
**The Mount Toft**
**Cambridge, CB23 2RL (GB)**

(72) Inventor: **Cotton, Symon D'Oyly**
**Great Gransden**
**Cambs SG19 3AF (GB)**

(74) Representative: **Beresford, Keith Denis Lewis et al**
**BERESFORD & Co.**
**16 High Holborn**
**London WC1V 6BX (GB)**

(54) **Method and apparatus for obtaining a measurement of sun damage**

(57)  Images of a first and a second area of skin of an individual are obtained (s1, s3) wherein one of said first and second areas of skin comprises an area of skin of the individual generally not exposed to sunlight. The images are then processed (s2,s4) to determine the concentration and distribution of one or more chromophores in the imaged areas of skin. A relative measure of skin damage is then calculated (s5) using the determined of concentrations and distributions of chromophores in said imaged areas of skin. The calculated measure for an area of skin can be based upon determined co-variances for the distribution of chromophores in the sampled areas. Differences between measurements for exposed and unexposed areas of skin provide the relative measure of sun damage.

Fig.1.

EP 1 917 908 A1

**Description**

The present application concerns methods for obtaining a measurement of skin damage. Embodiments of the invention are concerned with obtaining measurements of skin damage indicative primarily of damage arising due to exposure to sunlight.

The appearance of human skin is primarily determined by the concentration and distribution of three light absorbing molecules known as chromophores: melanin, haemoglobin, and collagen. Melanin gives the skin a brown colour, haemoglobin a red/pink colour and collagen contributes to the amount of light reflected from within the skin. As skin ages, the distribution of melanin, haemoglobin and collagen within the skin varies causing changes in the appearance of the skin.

It can be useful to classify the relative appearance of a sample of skin. To this end, prior art systems exist which contain a database of skin sample images ranked in some way. An individual's skin is sampled and then compared with the database. A skin score indicative of ranking of the appearance of the skin sample in the database most similar to the area being sampled in appearance is then output. Such a skin score can be used to identify for example the apparent age of a skin sample. The efficacy of cosmetics can also be determined by comparing the skin score of a skin sample with and without the presence of cosmetics.

A problem with pre-existing skin damage measurement systems is the need to acquire a representative database of the appearance of various skin samples. If the database is too small, accurate skin measurements are not possible. Even with a large database, concerns may exist as to how representative the skin samples within the database are compared with the general population at large. For these reasons an alternative system is desirable.

In accordance with one aspect of the present invention there is provided a method for obtaining a measurement of sun damage in accordance with claim 1.

In accordance with another aspect of the present invention there is provided an apparatus for obtaining a measurement of sun damage in accordance with claim 14.

Further aspects of the present invention will become apparent with reference to the accompanying drawings in which:

Figure 1 is a schematic block diagram of an apparatus in accordance with an embodiment of the present invention;
Figure 2 is a schematic cross sectional view through a layer of skin illustrating the structure of the skin and the interaction of that structure with incident light; and
Figure 3 is a flow diagram of a method of obtaining a measure of skin damage in accordance with an embodiment of the present invention.

Specific Embodiment

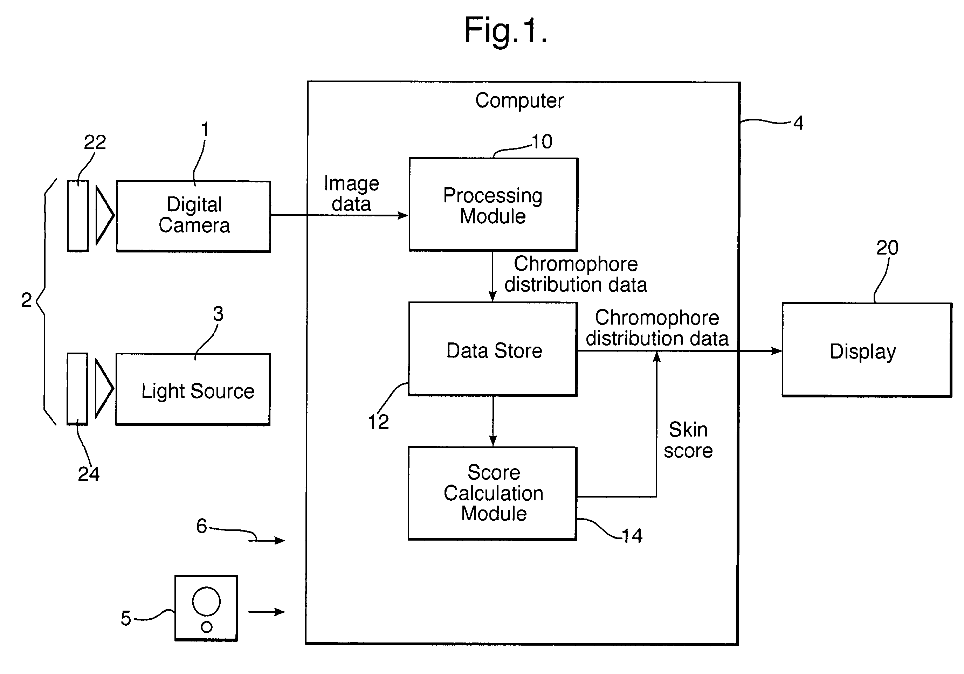

Figure 1 is a schematic block diagram with an embodiment of the present invention. In accordance with this embodiment, a digital camera 1 is provided which is arranged to obtain an image of an area of skin of an individual 2 illuminated by a light source 3. In this embodiment, the digital camera 1 comprises a camera operable to obtain red, green, blue and infra-red images of an area of skin being sampled. The images obtained by the digital camera 1 are then transmitted to a computer 4 which is configured by software either provided on a disc 5 or by receiving an electrical signal 6 via a communications network into a number of functional modules 10-14 operable to process the red, green, blue and infra-red image data received from the digital cameral 1 to generate an output image which is shown on a display 20.

In the present embodiment the functional modules 10, 12, 14 comprise a processing module 10 for converting red, green, blue and infra-red image data received from the digital camera 1 into chromophore distribution data which is then stored in a data store 12; and a score calculation module 14 which is arranged to process chromophore data stored in the data store 12 to generate a skin damage score when data for two separate areas of skin of the same individual have been sampled and stored. This score and images of the stored chromophore distributions are then passed to the display 20 where they can be shown to a user.

Interaction of the Light with the Skin

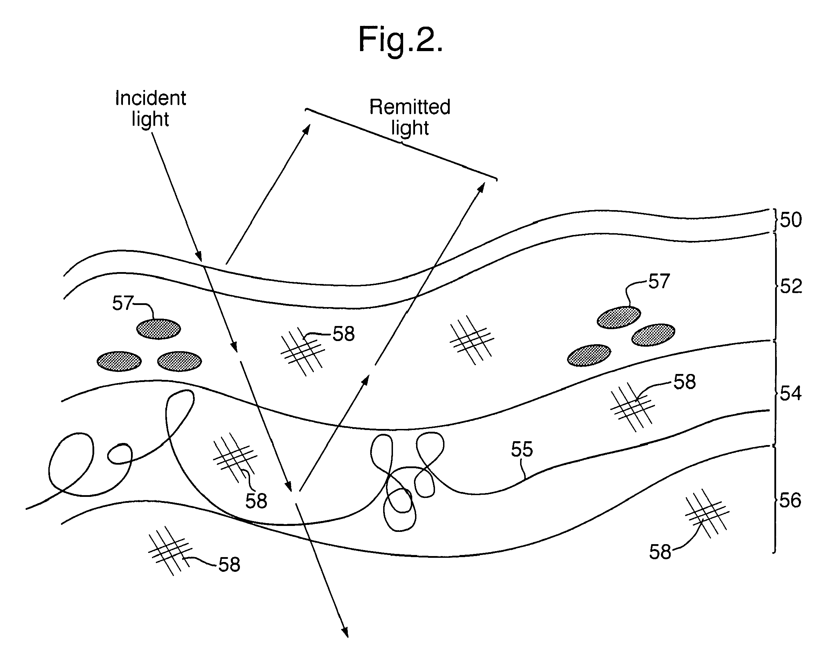

Prior to describing the detailed processing of the processing module 10 and the score calculation module 14 of the computer 4, the physical structure of skin and the interaction of skin with light will briefly be explained with reference to Figure 2.

As shown in Figure 2, skin has a layered structure comprising an outer cornified layer 50, the epidermis 52 and the dermis which itself can be divided into the papillary dermis 54 which contains the blood supply 55 for the skin and the reticular dermis 56.

When light is incident on the skin, much of the light is immediately reflected when coming into contact with the outer cornified layer 50. A proportion of the incident light does, however, pass through the cornified layer 50 and proceeds to interact with the constituents of the epidermis 52 and the papillary dermis 54.

As light passes through the epidermis 52 and the papillary dermis 54 the light is absorbed by various chromophores present in the skin. Most notably, chromophores such as haemoglobin present in blood in the blood vessels 55 in the papillary dermis, melanin a pigment produced by melanocytes 57 in the epidermis and collagen 58 a fibrous material present throughout the skin. By the time the incident light reaches the reticular dermis 56 the scattering of light is highly forward and for that reason the reticular dermis 56 can for all intense and purposes be considered returning no light.

In addition to chromophores present in the epidermis 52 and papillary dermis 54 absorbing various wavelengths, certain structures in the skin, most notably collagen 58, cause incident light to be reflected. The outward appearance of the skin can therefore be considered to be a mixture of light immediately reflected by the cornified layer 50 and the remitted light which has interacted with the chromophores present in the epidermis 52 and the papillary dermis 54.

In order to obtain measurements of the concentrations and distributions of chromophores in the papillary dermis 54 and epidermis 52, the effects of reflection of light directly by the cornified layer 50 is required to be removed so that a measure of the remitted light which has interacted with the chromophores present in the epidermis 52 and the papillary dermis 54 can be made.

Returning to Figure 1, in this embodiment a first polarising filter 22 is provided in front of the lens of the digital camera 1 and a second polarising filter 24 cross polarised with the first is provided in front the light source 3. As the interaction of light with collagen is such as to cause the light to lose its polarisation, by providing these filters 22,24, light from the light source 3 passing thorough the second polarising filter 38 which is reflected by the cornified layer 50 without interacting with other layers of the skin is caused to be filtered by the first polarising filter 22. The image data obtained by the digital camera 1 is thereby caused to be solely representative of light which is interactive with the structures of the epidermis 52 and papillary dermis 54 of an individual's skin.

Processing to Obtain a Measurement of Skin Damage

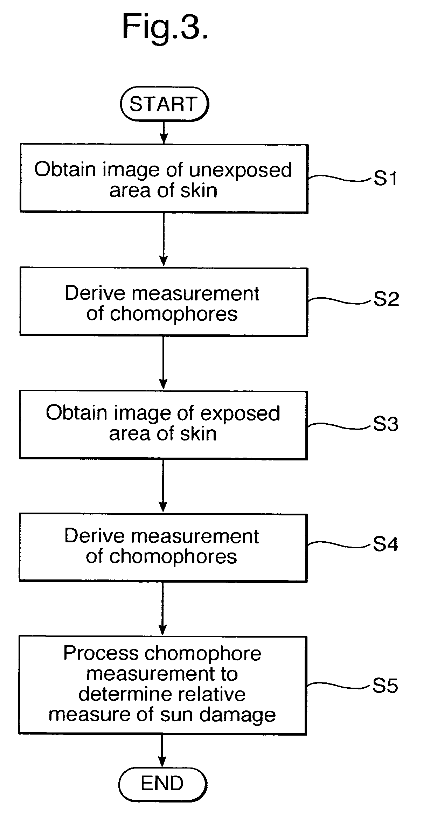

Referring to Figure 3 which is a flow diagram of a method in accordance with an embodiment of the present invention, initially (S1) an image is obtained by the digital camera 1 of a first area of skin of an individual 2 illuminated by the light source 3.

In this embodiment the image data generated by the digital camera 1 comprises red, green, blue and infra-red values ranging from zero to 255 for a large range of pixels where the red, green, blue and infra-red values are indicative of the extent of light received by a photo receptor within the camera 1 for each pixel in an image appears to be red, green, blue and infra-red respectively. Thus for example a completely cold black pixel will have red, green, blue and infra-red values of 0,0,0,0 and a hot completely bright white pixel will have values of 255,255,255,255.

In this embodiment of the present invention, the first area of skin of an individual 2 to be sampled is an area of skin not generally exposed to sunlight. Thus for example a suitable area of low exposure skin would be sampling an area of skin on the inner arm of an individual.

When an image of a first area of skin has been obtained by the camera 1, the image is then passed to the processing module 10 which converts (S2) the red, green, blue and infra-red image data for each pixel into chromophore distribution data identifying for each pixel in an image the apparent concentration of various chromophores giving rise to a particular appearance in an area of skin. The processing undertaken to derive chromophore measurements from red, green, blue and infra-red image values is such as has been previously discussed in Astron Clinica's earlier patent applications US 09/314751, US 09/760387, US 10/521638 and US 10/523158 and granted US patent US 6324417.

In this embodiment the processing module 10 is such to process red, green, blue and infra-red image data to generate three sets of chromophore distribution data. The first set of chromophore distribution data is an image indicative of the distribution and concentration of blood in area of skin being sampled. The second set of chromophore distribution data is an image illustrating the distribution and concentration of melanin in an area of skin being sampled, and the third set chromophore distribution data is an image indicative of the concentration and distribution of collagen in the area of skin being sampled.

Having derived measurements of chromophores in a first area of skin a second image (S3) is then obtained using the digital camera 1 of a second area of skin. In contrast to the first area of skin, this second area of skin is selected to be an area exposed to a high level of sunlight (e.g. an area on an individual's face). This second image is then processed (S4) in a similar way has as previously been described so as to generate further chromophore distribution data identifying distributions of blood, melanin and collagen which is also stored in the data store 12.

At this stage six sets of chromophore data will be stored within the data store 12. This chromophore data will comprise blood, melanin and collagen chromophore distribution data for a first area of skin, being an area of typically unexposed skin and blood, melanin and collagen distribution chromophore distribution data for an exposed area of skin.

Each set of chromophore distribution data will comprise a monochrome image of a skin sample where pixels in the image are associated with values ranging from 0 to 255 where pixels associated with low values are indicative of high concentrations of a chromophore and pixels associated with values are indicative of low concentrations of chromophores.

The score calculation module 14 then (S5) proceeds to process this stored chromophore distribution data to generate a measure of skin damage as will now be described.

In this embodiment, the calculated skin damage score is a value indicative of the relative disorder of the distribution of chromophores within skin samples. In young skin, generally chromophores are distributed within the skin in a relative homogenous way. As skin ages, typically due to sun exposure, the distribution of chromophores becomes increasingly disordered. Since this process occurs relatively slowly for areas of the skin which are not typically exposed to the sun, for example areas on the inner arm, a value of skin damage can be derived by making a comparison between a skin sample which is typically not exposed to sunlight and an area of skin sample which receives a high exposure to sunlight.

In this embodiment this is achieved by calculating a value based upon the sum of co-variance measures for the distributions of chromophores in an unexposed area of skin compared with the sum of co-variance measures for the distributions of chromophores in an exposed area of skin.

That is to say for each of the skin samples the following measure is calculated:

$$Abs\left(\frac{Std\left(Blood\right)}{Mean\left(Blood\right)}\right) + Abs\left(\frac{Std\left(Melanin\right)}{Mean\left(Melanin\right)}\right) + Abs\left(\frac{Std\left(Collagen\right)}{Mean\left(Collagen\right)}\right)$$

where abs () is an absolute value function, std () is a standard deviation function and mean () is a mean value function and Blood, Melanin and Collagen are the chromophore distribution image data for blood, melanin and collagen respectively for a sampled area of skin.

Typically in blood positive co-variances arise because the co-variance of blood vessels increases with sun exposure. Some negative co-variance of melanin can occur, for example if a depigmented region of exposed skin is imaged. Such areas are typically inverted freckles or Guttate Idiopathic Hypomelanosis. As such a negative co-variance is still indicative of damage and should be considered a positive value it is preferable for an absolute positive co-variance value to be determined and used when calculating the damage measure. It is therefore preferable when calculating the sum of co-variances to determine the sum of the absolute values of the calculated co-variances for the chromophore distributions.

A final relative skin damage measure can then be calculated by using the two measures for the two sample areas. Thus for example a skin damage measure for an exposed area of skin could be determined utilising the difference between the calculated co-variance measure for the exposed and unexposed skin samples. Image data illustrating the different chromophore distribution and a skin damage value either in the form of a figure or for example a slider displayed on the screen can then be generated and sent to the display 20.

Further Embodiments and Modifications

Although in the above described system a skin damage measure has been described based upon co-variances calculations for the distribution and concentration of three chromophores, it will be appreciated that measures based upon a different number of chromophores could be used.

In particular, it will be appreciated that a co-variance value based purely upon the distribution of blood and melanin could be utilised. The advantage of such a measure would be that images could be processed without requiring direct contact between a measuring device and an individual's skin so as to remove variations due to lighting and surface geometry when calculating the skin measurement. Additionally, measurements of blood and melanin could be obtained by processing conventional red, green and blue image data rather than red, green, blue and infra-red image data as described in the above embodiment. Thus for this reason a conventional digital colour camera could be used to obtain images of skin samples rather than a camera arranged to obtain, red, green, blue and infra-red images.

It will also be appreciated that different functions could be utilised to calculate the outputted skin damage measure. Thus for example the contributions to the measure due to the co-variances of various chromophores could be scaled to increase or decrease the relative contribution of the co-variances for the different chromophores.

Further it will be appreciated that in order to avoid co-variances which are not truly representative of a skin sample and various pre processing steps could be undertaken. Thus for example when calculating a co-variance rather than utilising a determined mean and standard deviation for an entire image, a selected portion of an image could be utilised so as to avoid areas at for example the edge of an image which might not be representative. Further in order to avoid the distorting effects of very low mean values for an image, in the case of images where the calculated mean was below a certain limit, a prefixed minimum value could be used instead of the mean when calculating a co-variance value.

When calculating the score to be displayed the score could be scaled to a value between a maximum value and a minimum value where the maximum and minimum value represents calculated maximum and minimum values for the range of skin samples.

Although the embodiments of the invention described with reference to the drawings comprise computer apparatus and processes performed in computer apparatus, the invention also extends to computer programs, particularly computer programs on or in a carrier, adapted for putting the invention into practice. The program may be in the form of source or object code or in any other form suitable for use in the implementation of the processes according to the invention. The carrier can be any entity or device capable of carrying the program.

For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal which may be conveyed via electrical or optical cable or by radio or other means.

When a program is embodied in a signal which may be conveyed directly by a cable or other device or means, the carrier may be constituted by such cable or other device or means.

Alternatively, the carrier may be an integrated circuit in which the program is embedded, the integrated circuit being adapted for performing, or for use in the performance of, the relevant processes.

**Claims**

1. A method for obtaining a measurement of sun damage comprising:

   obtaining (S1,S3) an image of a first and a second area of skin of an individual, wherein one of said first and second areas of skin comprises an area of skin of the individual generally not exposed to sunlight;
   processing(S2,S4) said images to determine the concentration and distribution of one or more chromophores in said first and second area of skin of said individual; and
   processing (S5) said determinations of concentrations and distributions of chromophores in said first and second areas of skin to generate a relative measure of skin damage.

2. A method in accordance with claim 1 wherein processing (S5) said determinations of concentrations and distributions of chromophores in said first and second areas of skin to generate a relative measure of skin damage comprises:

   calculating a covariance measure for the concentrations and distributions of one or more chromophores in said first and second areas of skin; and
   generating a relative measure of skin damage utilising said calculated covariance for the concentrations and distributions of one or more chromophores in said first and second areas of skin.

3. A method in accordance with claim 2 wherein calculating a covariance measure comprises:

   determining for a selected area of skin for which an image has been obtained the standard deviation of chromophore concentrations in said area of skin divided by a mean chromophore concentration for said one or more chromophores.

4. A method in accordance with claim 3 wherein calculating a covariance measure comprises:

   determining whether a mean chromophore concentration for said one or more chromophores is less than a threshold value;
   if said mean chromophore concentration for a chromophore is greater than said threshold value, determining for a selected area of skin for which an image has been obtained the standard deviation of chromophore concentrations in said area of skin divided by a mean chromophore concentration for said chromophore; and
   if said mean chromophore concentration is less than said threshold value, determining for said selected area of skin for which an image has been obtained the standard deviation of chromophore concentrations in said area of skin divided by a predetermined value.

5. A method in accordance with claim 3 or 4, wherein said one or more chromophores comprise a plurality of chromophores, wherein utilising said calculated co-variances comprises summing said calculated co-variances for an area of skin to generate for a damage measure for an area of skin and utilising the generated damage measure for said first and second areas to generate a relative measure of skin damage relative to the damage measure for said an area of skin of the individual generally not exposed to sunlight.

6. A method in accordance with claim 5 wherein summing said calculated co-variances comprises summing said co-variances each scaled by a scaling factor.

7. A method in accordance with claim 5 or claim 6 wherein summing said calculated co-variances comprises summing the absolute values of said co-variances.

8. A method in accordance with any proceeding claim further comprising outputting said generated relative measure of skin damage.

9. A method in accordance with claim 8 wherein outputting said generated relative measure of skin damage comprises generating and outputting a display including a slider indicating said generated relative measure of skin damage.

10. A method in accordance with claim 9 wherein said generated display includes a representation of said determined the concentration and distribution of one or more chromophores in at least one of said first and second area of skin of said individual.

11. A method in accordance with any preceding claim wherein said one or more chromophores comprise haemoglobin.

12. A method in accordance with any preceding claim wherein said one or more chromophores comprise melanin.

13. A method in accordance with any preceding claim wherein said one or more chromophores comprise collagen.

14. An apparatus for obtaining a measurement of sun damage comprising:

   means (1,22) for obtaining an image of a first and a second area of skin of an individual, wherein one of said first and second areas of skin comprises an area of skin of the individual generally not exposed to sunlight;
   means (10) for processing obtained images to determine the concentration and distribution of one or more chromophores in said first and second area of skin of said individual; and
   means (14) for generating a relative measure of skin damage based upon determinations of concentrations and distributions of chromophores in said first and second areas of skin.

15. An apparatus in accordance with claim 14 wherein said means (14) for generating a relative measure of skin damage is operable to:

   calculate a covariance measure for determined concentrations and distributions of one or more chromophores in said first and second areas of skin; and
   generate a relative measure of skin damage utilising said calculated covariance for the concentrations and distributions of one or more chromophores in said first and second areas of skin.

16. An apparatus in accordance with claim 15 wherein calculating a covariance measure comprises:

   determining for a selected area of skin for which an image has been obtained the standard deviation of chromophore concentrations in said area of skin divided by a mean chromophore concentration for said one or more chromophores.

17. An apparatus in accordance with claim 16 wherein calculating a covariance measure comprises:

   determining whether a mean chromophore concentration for said one or more chromophores is less than a threshold value;
   if said mean chromophore concentration for a chromophore is greater than said threshold value, determining for a selected area of skin for which an image has been obtained the standard deviation of chromophore concentrations in said area of skin divided by a mean chromophore concentration for said chromophore; and

if said mean chromophore concentration is less than said threshold value, determining for said selected area of skin for which an image has been obtained the standard deviation of chromophore concentrations in said area of skin divided by a predetermined value.

18. An apparatus in accordance with claim 16 or 17, wherein said means (10) for processing obtained images to determine the concentration and distribution of one or more chromophores in said first and second area of skin of said individual is operable to determine the concentration and distribution of a plurality of chromophores, and wherein said means for generating a relative measure of skin damage is operable to sum calculated co-variances for a plurality of chromophore to generate a damage measure for an area of skin and utilise the generated damage measure for said first and second areas to generate a relative measure of skin damage relative to the damage measure for said an area of skin of the individual generally not exposed to sunlight.

19. An apparatus in accordance with claim 18 wherein summing said calculated co-variances comprises summing said co-variances each scaled by a scaling factor.

20. An apparatus in accordance with claim 18 or claim 19 wherein summing said calculated co-variances comprises summing the absolute values of said co-variances.

21. An apparatus in accordance with any of claims 14-20 further comprising means for outputting said generated relative measure of skin damage.

22. An apparatus in accordance with claim 21 wherein said means for outputting said generated relative measure of skin damage is operable to generate and output a display including a slider indicating said generated relative measure of skin damage.

23. An apparatus in accordance with claim 22 wherein said generated display includes a representation of said determined concentrations and distributions of one or more chromophores in at least one of said first and second area of skin of said individual.

24. An apparatus in accordance with any of claims 14-23 wherein said means (10) for processing is operable to determine the concentration and distribution haemoglobin in said first and second area of skin of said individual.

25. An apparatus in accordance with any of claims 14-24 wherein said means (10) for processing is operable to determine the concentration and distribution melanin in said first and second area of skin of said individual.

26. An apparatus in accordance with any of claims 14-25 wherein said means (10) for processing is operable to determine the concentration and distribution collagen in said first and second area of skin of said individual.

27. A storage medium storing computer implementable instructions operable to cause a programmable computer to perform a method in accordance with any of claims 1-13.

28. A storage medium in accordance with claim 27 comprising a computer disc.

29. A storage medium in accordance with claim 27 comprising an electrical signal within a communications network.

Fig.1.

# Fig.2.

Incident light

Remitted light

57

58

57

58

58

54

55

58

56

58

58

EP 1 917 908 A1

# Fig.3.

```
        ( START )
            │
            ▼
┌───────────────────────┐
│  Obtain image of unexposed  │——— S1
│      area of skin           │
└───────────────────────┘
            │
            ▼
┌───────────────────────┐
│    Derive measurement       │——— S2
│      of chomophores         │
└───────────────────────┘
            │
            ▼
┌───────────────────────┐
│    Obtain image of exposed  │——— S3
│      area of skin           │
└───────────────────────┘
            │
            ▼
┌───────────────────────┐
│    Derive measurement       │——— S4
│      of chomophores         │
└───────────────────────┘
            │
            ▼
┌───────────────────────┐
│   Process chomophore        │
│    measurement to           │——— S5
│   determine relative        │
│  measure of sun damage      │
└───────────────────────┘
            │
            ▼
        (  END  )
```

**European Patent Office**

**EUROPEAN SEARCH REPORT**

Application Number

EP 07 25 4185

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 1 314 395 A (OREAL [FR]) 28 May 2003 (2003-05-28) <br><br> * paragraph [0012] - paragraph [0043] * <br> ----- | 1-3,8, 11-16, 21,24-26 | INV. A61B5/103 |
| Y | US 2005/251049 A1 (CANE MICHAEL R [GB] ET AL) 10 November 2005 (2005-11-10) <br><br> * paragraph [0178] - paragraph [0198] * <br> * claims 1,24 * <br> ----- | 1-3,8, 11-16, 21,24-26 | |
| A | US 2005/030372 A1 (JUNG BYUNGJO [US] ET AL) 10 February 2005 (2005-02-10) <br> * the whole document * <br> ----- | 1-29 | |

TECHNICAL FIELDS SEARCHED (IPC)

A61B

The present search report has been drawn up for all claims

| Place of search | Date of completion of the search | Examiner |
|---|---|---|
| Munich | 7 February 2008 | Rivera Pons, Carlos |

EPO FORM 1503 03.82 (P04C01)

## ANNEX TO THE EUROPEAN SEARCH REPORT
## ON EUROPEAN PATENT APPLICATION NO.

EP 07 25 4185

This annex lists the patent family members relating to the patent documents cited in the above-mentioned European search report.
The members are as contained in the European Patent Office EDP file on
The European Patent Office is in no way liable for these particulars which are merely given for the purpose of information.

07-02-2008

| Patent document cited in search report | | Publication date | Patent family member(s) | | Publication date |
|---|---|---|---|---|---|
| EP 1314395 | A | 28-05-2003 | JP<br>US | 2003220037 A<br>2003099383 A1 | 05-08-2003<br>29-05-2003 |
| US 2005251049 | A1 | 10-11-2005 | NONE | | |
| US 2005030372 | A1 | 10-02-2005 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 09314751 B
- US 09760387 B
- US 10521638 B
- US 10523158 B
- US 6324417 B